# EUROPÄISCHE PATENTSCHRIFT

(11) **EP 2 838 465 B1**
(45) Veröffentlichungstag und Bekanntmachung des Hinweises auf die Patenterteilung: **27.06.2018**
(21) Anmeldenummer: 13716813.4
(22) Anmeldetag: 18.04.2013
(51) Int. Cl.: A61C 13/00, A61C 13/36

(54) **VERFAHREN ZUM HERSTELLEN EINES KÜNSTLICHEN GEBISSES**
METHOD FOR PRODUCING DENTURES
PROCÉDÉ DE PRODUCTION D'UN DENTIER

(30) Priorität: 19.04.2012 DE 102012007706
(43) Veröffentlichungstag der Anmeldung: 25.02.2015
(73) Patentinhaber: Kulzer GmbH, 63450 Hanau (DE)
(72) Erfinder: BEYER, Mario, 61350 Bad Homburg (DE); BÖHM, Uwe, 63456 Hanau (DE)
(74) Vertreter: Bendele, Tanja
(86) Internationale Anmeldenummer: PCT/EP2013/058111
(87) Internationale Veröffentlichungsnummer: WO 2013/156572

(56) Entgegenhaltungen:
- WO-A2-2012/021816
- DE-A1- 3 715 106
- DE-A1-102009 056 752

## Beschreibung

Die Erfindung betrifft ein Verfahren zum Herstellen eines künstlichen Gebisses.
Im Stand der Technik sind sogenannte Teil- oder Totalprothesen bekannt, die ggf. reversibel aus dem Kiefer entfernt werden können. Im Allgemeinen werden die Teil- oder Totalprothesen durch Unterdruck, Adhäsion oder Schrauben mit dem Kiefer bzw. einem im Kiefer eingebrachten Implantat verbunden. Dabei weisen solche Prothesen eine Basis auf, die auf dem Zahnfleisch zu liegen kommt. Auf jener Basis - auch als Basiselement bezeichnet - werden einzelne Zähne aufgebracht, welche die natürlichen Zähne ersetzen. Als problematisch im Stand der Technik hat sich dabei insbesondere die Positionierung und Einbringung der künstlichen Zähne in das Basiselement herausgestellt. Die verwendeten künstlichen Zähne sind vorkonfektioniert. Insofern müssen die künstlichen Zähne auf die genutzten Längen gekürzt werden. Um eine Anpassung an die individuell benötigten Zahnlängen und -breiten sicherzustellen, ist es zum einen möglich, eine sehr große Anzahl unterschiedlicher künstlicher Zähne herzustellen, welche die meistgenutzten Längen bzw. Breiten abbilden. Dies ist allerdings sehr teuer und aufwändig. Alternativ ist ein individuelles Abschleifen der einzelnen Zahnrohlinge jener künstlichen Zähne möglich, die später in das Basiselement eingesetzt werden. Bei dieser Art des Verfahrens hat sich allerdings herausgestellt, dass die erreichte Passgenauigkeit zu gering ist. Darüber hinaus besteht die Gefahr, dass die mehrlagig aufgebauten Zahnrohlinge derart beschädigt werden, dass die Haltbarkeit des so hergestellten Gebisses deutlich reduziert wird.

Der Dokument WO 2012/021816 offenbart ein Verfahren zum Herstellen eines künstlichen Gebisses, wobei der untere Teil der Zahnrohlinge mit Hilfe einer Negativform verkürzt wird.

Die Aufgabe der vorliegenden Erfindung liegt darin, die oben genannten Nachteile zu überwinden. Insbesondere soll ein Verfahren offenbart werden, mit dem es möglich ist, künstliche Gebisse für eine Vielzahl von Menschen herzustellen, ohne dabei Gefahr zu laufen, dass bei der individuellen Anpassung der Zahnrohlinge diese in einem Maße beschädigt werden können, dass die Dauerhaftigkeit des Gebisses nachhaltig geschwächt wird.

Zur Lösung dieser Aufgabe wird ein Verfahren zum Herstellen eines künstlichen Gebisses mit den Merkmalen des unabhängigen Anspruchs 1. Weiterhin werden zur Lösung dieser Aufgabe eine Negativform und ein künstliches Gebiss vorgeschlagen. Vorteilhafte Weiterbildungen der Erfindung, welche einzeln oder in Kombination realisierbar sind, sind in den abhängigen Ansprüchen dargestellt. Merkmale und Details, die in Zusammenhang mit dem Verfahren oder der Negativform oder dem künstlichen Gebiss beschrieben werden, gelten dabei auch jeweils für die anderen Ausgestaltungen bzw. Erscheinungsformen der Erfindung.
Zusammenfassend werden folgende Ausführungsformen im Rahmen der vorliegenden Erfindung als besonders bevorzugt vorgeschlagen:
Ausführungsform 1: Verfahren zum Herstellen eines künstlichen Gebisses wobei das künstliche Gebiss ein Basiselement aufweist, welches wenigstens zwei Zahnrohlinge hält, mit den Schritten:
   a. Erstellen des digitalen Abbildes einer Zahnsituation,
   b. computergestütztes Erzeugen eines digitalen Modelles des künstlichen Gebisses auf Basis des digitalen Abbildes,
      - unter Verwendung und Auswahl von digitalen Darstellungen von vorgefertigten Zahnrohlingen,
      - wobei die vorgefertigten Zahnrohlinge eine definierte Längenabmessung aufweisen,
   c. Berechnung eines Längendifferenz-Datensatzes, erstellt aus der definierten Längenabmessung und einer Einsetztiefe der wenigstens zwei vorgefertigten Zahnrohlinge in dem Basiselement,
   d. Aufbauen eines digitalen Bildes einer Negativform, bei der die wenigstens zwei vorgefertigten Zahnrohlinge das Positiv bilden, wobei die Negativform die Position der wenigstens zwei vorgefertigten Zahnrohlinge auf dem Basiselement codiert,
   e. Maschinelles Erstellen der Negativform mittels des digitalen Bildes der Negativform,f. Einfügen der vorgefertigten Zahnrohlinge in die Negativform,
   g. computergestütztes maschinelles Verkürzen der Längenabmessung der vorgefertigten Zahnrohlinge von einer basalen Seite auf Basis des Längendifferenz-Datensatzes zur Herstellung der verkürzten Zahnrohlinge,
   h. Verbinden des Basiselementes und der verkürzten Zahnrohlinge zu dem künstlichen Gebiss.
Ausführungsform 2: Verfahren nach der vorhergehenden Ausführungsform, dadurch gekennzeichnet, dass zum Erstellen der Negativform ein auftragendes computergestütztes Herstellungsverfahren genutzt wird, insbesondere dass das auftragende computergestützte Herstellungsverfahren ausgewählt ist aus der Gruppe bestehend aus: Rapid Prototyping, 3D-Lithografie, SLM (selektives Laserschmelzen), 3D-Stereolithgrafie, 3D-Inkjet, FDM (fused deposition modelling) und 3D-Laserlithographie oder mindestens zwei davon.
Ausführungsform 3: Verfahren nach wenigstens einer der vorhergehenden Ausführungsformen, dadurch gekennzeichnet, dass die vorgefertigten Zahnrohlingen kraft- und/oder form- und/oder stoffschlüssig in der Negativform gehalten werden.
Ausführungsform 4: Verfahren nach wenigstens einer der vorhergehenden Ausführungsformen, dadurch gekennzeichnet, dass das Verfahren den Schritt aufweist:
   - Erstellen eines Befestigungsmittels zur Verbindungen der verkürzten Zahnrohling mit dem Basisteil, wobei das Befestigungsmittel ein erstes Codierungsmittel aufweist, welches eine räumlich eindeutige Positionierung des verkürzten Zahnrohlings in dem Basisteil ermöglicht.
Ausführungsform 5: Verfahren nach wenigstens einer der vorhergehenden Ausführungsformen, dadurch gekennzeichnet, dass die Negativformen mit wenigstens einem zweiten Codierungsmittel versehen wird, welches eine räumlich eindeutige Positionierung des vorgefertigten Zahnrohlings in der Negativform ermöglicht.
Ausführungsform 6: Verfahren nach der vorhergehenden Ausführungsform 1, dadurch gekennzeichnet, dass in Schritt b) die Auswahl aus einer Menge von digitalen Darstellungen von vorgefertigten Zahnrohlingen erfolgt, um eine kleinstmögliche Abweichung einer geometrischen Form der vorgefertigten Zahnrohlingen von der Zahnsituation zu erzielen.
Ausführungsform 7: Verfahren nach der vorhergehenden Ausführungsform 1, dadurch gekennzeichnet, dass in Schritt b) die Auswahl aus einer Menge von digitalen Darstellungen von vorgefertigten Zahnrohlingen erfolgt, um eine kleinstmögliche Abweichung einer geometrischen Form der vorgefertigten Zahnrohlingen von einer vordefinierten Zahnanordnung zu erreichen.
Ausführungsform 8: Verfahren nach wenigstens einer der vorhergehenden Ausführungsformen, dadurch gekennzeichnet, dass in Schritt c) die ausgewählten digitalen Darstellungen vorgefertigter Zahnrohlinge als ein Zahnbogen angeordnet werden.
Ausführungsform 9: Verfahren nach wenigstens einer der vorhergehenden Ausführungsformen, dadurch gekennzeichnet, dass in Schritt c) ausgehende von der Zahnsituation und/oder einer vordefinierten Zahnanordnung die ausgewählten, digitalen Darstellungen der Zahnrohlinge an einer Kronenseite entlang einer Zahnlinie angeordnet werden.
Ausführungsform 10: Verfahren nach wenigstens einer der vorhergehenden Ausführungsformen, dadurch gekennzeichnet, dass nach dem Schritt b) das Verfahren den Schritt aufweist: Anpassen einer Höhenposition der digitalen Darstellungen der vorgefertigten Zahnrohlinge in dem digitalen Modell des künstlichen Gebisses.
Ausführungsform 11: Verfahren nach wenigstens einer der vorhergehenden Ausführungsformen, dadurch gekennzeichnet, dass das Verfahren den Schritt aufweist: computergestütztes Teilen des digitalen Modelles des künstlichen Gebisses in ein digitales Abbild des Basiselementes und ein digitales Ebenbild der wenigstens zwei Zahnrohlinge.
Ausführungsform 12: Verfahren nach der vorhergehenden Ausführungsform 11, dadurch gekennzeichnet, dass das digitale Ebenbild ein Negativ für die Negativform bildet.
Ausführungsform 13: Verfahren nach der vorhergehenden Ausführungsform 11, dadurch gekennzeichnet, dass das Verfahren den Schritt aufweist: Maschinelles Fertigen des Basiselementes auf Basis des digitalen Abbildes des Basiselementes.
Ausführungsform 14: Verfahren nach wenigstens einer der vorhergehenden Ausführungsformen, dadurch gekennzeichnet, dass die Negativform mit wenigstens einem Positionierungselement versehen wird, um eine Positionierung der Negativform beim maschinellen Verkürzen der Längenabmessung zu ermöglichen.
Ausführungsform 15: Verfahren nach wenigstens einer der vorhergehenden Ausführungsformen, dadurch gekennzeichnet, dass das Basisteil ein Material aufweist oder aus einem Material besteht, ausgewählt aus der Gruppe: Polyethylen, Polypropylen, Polystyrol, Poly(methylmethacrylat), AcryINitril-Butadien-Styrol-Copolymerisat, Epoxid oder Acrylate.
Ausführungsform 16: Verfahren nach wenigstens einer der vorhergehenden Ausführungsformen, dadurch gekennzeichnet, dass das Basisteil und die verkürzten Zahnrohlinge kraft- und/oder form- und/oder stoffschlüssig verbunden werden.
Ausführungsform 17: Verfahren nach wenigstens einer der vorhergehenden Ausführungsformen, dadurch gekennzeichnet, dass ein Befestigungsmittels zur Verbindung des Basisteils und der verkürzten Zahnrohlinge dient, wobei das Befestigungsmittel ausgewählt ist aus der Gruppe bestehend aus: einem Kleber, einem mechanischen Verbindungsmittel, einer Schraube, einem Gewinde, einem Stift, einem Bajonettverschluss oder mindestens zwei davon.
Ausführungsform 18: Verfahren nach wenigstens einer der vorhergehenden Ausführungsformen, dadurch gekennzeichnet, dass ein abtragendes und/oder auftragendes computergestütztes Herstellungsverfahren genutzt wird in dem Verfahrensschritt: maschinelles Erstellen der Negativform und/oder maschinelles Fertigen des Basiselementes.
Ausführungsform 19: Verfahren nach der vorhergehenden Ausführungsform 1 oder 18, dadurch gekennzeichnet, dass das auftragende computergestützte Herstellungsverfahren ausgewählt ist aus der Gruppe bestehend aus: Rapid Prototyping, 3D-Laserlithographie, 3D-Lithografie, SLM (selektives Laserschmelzen), 3D-Stereolithgrafie, 3D-Inkjet, FDM (fused deposition modelling) und 3D-Laserlithographie oder mindestens zwei davon.
Ausführungsform 20: Verfahren nach wenigstens einer der vorhergehenden Ausführungsformen, dadurch gekennzeichnet, dass zum Formen des digitalen Modelles des künstlichen Gebisses ein digitalen Abbildes einer Zahnsituation verwendet wird, das die Form eines Zahnfleischareals eines Patienten wiedergibt.
Ausführungsform 21: Verfahren nach wenigstens einer der vorhergehenden Ausführungsformen, dadurch gekennzeichnet, dass das digitale Modelle durch Abscannen eines Abdruckmodells des Zahnfleischareals oder durch Abscannen des Zahnfleischareals eines Patienten gewonnen wird.
Ausführungsform 22: Computerlesbarer Datenträger der Instruktionen umfasst, die einen Computer veranlassen, ein Verfahren nach einem der vorhergehenden Ausführungsformen auszuführen.
Ausführungsform 23: Computer mit einem computerlesbaren Datenträger nach der vorhergehenden Ausführungsform 22.
Der Kern des erfindungsgemäßen Verfahrens besteht in der Herstellung einer Negativform. Für diese Negativform bilden die im Kiefer des Patienten angeordneten natürlichen Zähne und/oder die erwünschte Anordnung der künstlichen Zahnrohlinge im Mund das Positiv. Insofern weist diese Negativform sämtliche Informationen über die Position und Länge der Zähne im Kiefer des Patienten auf. Erfindungsgemäß ist nunmehr vorgesehen, dass in die Negativform vorproduzierte Zahnrohlinge eingesetzt werden und mit einem abtragenden Verfahren auf die benötigte Länge angepasst werden. Die Negativform schützt somit die Oberfläche der künstlichen Zahnrohlinge und stellt gleichfalls sicher, dass jene auf die gewünschte Länge angepasst werden können. Die Besonderheit besteht dabei darin, dass nicht individuell einzelne Zähne angepasst, sondern gleich eine Mehrzahl von künstlichen Zähnen parallel bearbeitet werden können. Gleichzeitig sorgt die feste Halterung der vorgefertigten Zahnrohlinge in der Negativform für eine erhöhte Präzision der zur erzielenden Zahnlänge im Vergleich zu bekannten Verfahren. Gleichzeitig kann im Rahmen dieses Verfahrens sichergestellt werden, dass etwaige Mindestlängen der Zahnrohlinge nicht unterschritten werden, um so deren mehrlagigen Aufbau nicht zu zerstören.

Im Rahmen der Erfindung sollen die Begriffe "Zahnrohling" oder "vorgefertigter Zahnrohling" auf ein Element verweisen, welches in seiner Form und äußeren Anmutung einem natürlichen Zahn entspricht. Solcherart Zahnrohlinge werden im Allgemeinen aus Kunststoffen hergestellt. Bei hochwertigen Zahnrohlingen findet ein mehrschichtiger Aufbau statt. Die Schichten werden dabei im Heißpolymerisationsverfahren einzeln auspolymerisiert: Zuerst der Dentinkern, dann die Schneide- und Schmelzschicht. Jedes der genannten Elemente des Zahnrohlings kann aus einem anderen Kunststoff aufgebaut werden. Durch den mehrschichtigen Aufbau ist es möglich, die technischen Eigenschaften schichtspezifisch anzupassen. So muss die der hohen Kaubelastung ausgesetzte Schneideschmelzschicht besonders abrasionsfest sein. Hingegen sollte die basale Halsschicht für einen optimalen Verbund zum Prothesenkunststoff - dem Kunststoff des Basiselements - weniger dicht vernetzt und leichter ablösbar sein.

Unter dem Begriff "basal" wird erfindungsgemäß die Wurzelseite der Zahnrohlinge verstanden. Ein verkürzter Zahnrohling ist der basal bearbeitete Zahnrohling bzw. vorgefertigte Zahnrohling.

Das erfindungsgemäße Verfahren dient zur Herstellung von künstlichen Gebissen. Unter dem Begriff "künstliches Gebiss" sollen insbesondere Teilprothese und Totalprothesen verstanden werden. Eine Teilprothese kann in unterschiedlichen Ausführungen hergestellt werden. Die einfachste Ausführung besteht aus einer Kunststoffbasis - das Basiselement -, den zu ersetzenden Zähnen und gebogenen Halte- und Stützelementen. Die Prothese kann nötigenfalls durch einen eingearbeiteten Draht oder Bügel verstärkt werden. Als Legierung für Teile des Basiselementes können Chrom-Cobalt-Molybdän oder Titan eingesetzt werden, da diese besonders gewebeverträglich sind. Auf dieser Basis werden dann Kunststoff und Zähne aufgebaut. Sind in einem Kiefer alle Zähne verloren gegangen, so bleibt oftmals nur eine Totalprothese als Lösung übrig. Diese findet durch Unterdruck, Schrauben und/oder Adhäsion ihren Halt am Kiefer.

Erfindungsgemäß wird vorgeschlagen, dass zur Erstellung der Negativform ein auftragendes, computergestütztes Herstellungsverfahren genutzt wird. Dieses auftragende, computergestützte Herstellungsverfahren wird vorteilhafterweise ausgewählt aus der Gruppe bestehend aus: Rapid Prototyping, 3D-Laserlithographie, 3D-Lithografie, SLM (selektives Laserschmelzen), 3D-Stereolithgrafie, 3D-Inkjet, FDM (fused deposition modelling) oder 3D-Laserlithographie. Die genannten auftragenden Herstellungsverfahren weisen eine Mehrzahl von Vorteilen auf. Es ist mit ihnen leicht möglich, auf Basis eines digitalen Abbildes einer Zahnsituation eine Negativform herzustellen. Entsprechende Rechnerprogramme können das digitale Abbild der Zahnsituation innerhalb kurzer Zeit in eine Negativform umrechnen. Darüber hinaus sind die produktionsbedingten Abweichungen von dem digitalen Abbild der Negativform gering. Präzisionen in der Größenordnung von +/- 20 µm, insbesondere +/- 10 µm lassen sich mit den genannten Herstellungsverfahren realisieren. Somit ist sichergestellt, dass die Halterung der vorgefertigten Zahnrohlinge in der Negativform nur mit einem Fehler von +/- 20 µm, insbesondre +/- 10 µm versehen ist. Jenes hat dann einen positiven Einfluss auf die spätere Halterung der gekürzten Zahnrohlinge in dem künstlichen Gebiss sowie den Tragekomfort des künstlichen Gebisses für den Patienten.

Vorteilhafterweise werden die vorgefertigten Zahnrohlinge kraft- und/oder form- und/oder stoffschlüssig in der Negativform gehalten. Ein wesentlicher Aspekt ist dabei, dass die vorgefertigten Zahnrohlinge reversibel lösbar, aber positionsstabil in der Negativform angeordnet sind. Als vorteilhaft haben sich entsprechende stoffschlüssige Verbindungen mittels Klebern oder Magneten herausgestellt, die ein entsprechendes Positionieren und Halten der Zahnrohlinge ermöglichen. Weiterhin ist es vorteilhaft, wenn die Negativform mit wenigstens einem zweiten Codierungsmittel versehen wird, welches eine räumlich eindeutige Positionierung des vorgefertigten Zahnrohlings in der Negativform ermöglicht. Als zweites Codierungsmittel kann etwa ein Vorsprung dienen, welcher in ein entsprechendes Gegenelement in dem vorgefertigten Zahnrohling eingreift bzw. mit diesem kommuniziert. Dadurch wird eine eindeutige Anordnung der vorgefertigten Zahnrohlinge in der Negativform sichergestellt. Es kann so insbesondere kein Vertauschen der Vorder- bzw. Rückseite der Zahnrohlinge auftreten.

Weiterhin hat es sich als vorteilhaft erwiesen, wenn das Basiselement mit einem Befestigungsmittel versehen wird. Dieses Befestigungsmittel dient zur Halterung der verkürzten Zahnrohlinge in dem Basisteil. Darüber hinaus ist das Befestigungsmittel derart ausgestaltet, dass es ein erstes Codierungsmittel aufweist, welche eine räumlich eindeutige Anordnung des verkürzten Zahnrohlings in dem Basisteil ermöglicht. Es soll sichergestellt werden, dass die verkürzten Zahnrohlinge nur in einer eindeutigen Art und Weise in das Basisteil eingeführt und dort kraft- und/oder form- und/oder stoffschlüssig angeordnet werden können. Vorteilhafterweise ist das Befestigungsmittel und/oder das Basisteil derart ausgestaltet, dass der verkürzte Zahnrohling mit einer Positionsgenauigkeit von +/- 20 µm, vorteilhafterweise +/- 10 µm, in dem Basisteil angeordnet werden kann. Dabei kann das Befestigungsmittel viele Ausgestaltungsvarianten, wie etwa einen Bajonettverschluss, ein Gewinde oder einen Stift aufweisen, die die räumliche Anordnung der verkürzten Zahnrohlinge im Basiselement sicherstellen.

Im Rahmen der Erfindung findet in einem Schritt a) ein Erstellen des digitalen Abbildes einer Zahnsituation statt. Ein entsprechendes digitales Abbild kann beispielsweise mit einem Interoralscanner direkt am Patienten erstellt werden. Alternativ ist es möglich, dass die Zahnsituation des Patienten mit einer Abdruckmasse abgenommen und dann in ein Positiv realisiert wird. Entsprechende Positive können mit Industriescannern in hoher Präzision digital abgebildet werden. Basierend auf diesem digitalen Abbild der Zahnsituation findet ein computergestütztes Erzeugen eines digitalen Modells des künstlichen Gebisses statt. Auf dem digitalen Modell aufbauend wird dann unter Verwendung entsprechender digitaler Dateien bzw. Modelle ein künstliches Gebiss individuell für den Patienten erstellt. Erfindungsgemäß ist dabei vorgesehen, dass eine Verwendung und Auswahl von digitalen Darstellungen von vorgefertigten Zahnrohlingen genutzt wird. Ausgangspunkt ist dabei der Gedanke, dass nur eine vergleichsweise geringe Zahl von vorgefertigten Zahnrohlingen genutzt werden soll. Diese werden digital gescannt und dem Ersteller des digitalen Modells des künstlichen Gebisses in einer Datenbank zur Verfügung gestellt. Durch ein entsprechendes Auswahlverfahren kann der Verwender die digitalen Darstellungen der vorgefertigten Zahnrohlinge nutzen, um das digitale Modell des künstlichen Gebisses herzustellen. Dabei finden Kriterien wie Größe, Breite und Farbe Eingang bei der Auswahl der zu nutzenden vorgefertigten Zahnrohlinge. Die Längenabmessungen der vorgefertigten Zahnrohlinge sind im Allgemeinen nicht kompatibel mit den tatsächlich genutzten Längen derjenigen Zahnrohlinge, die in das tatsächliche Gebiss eingebaut werden sollen. Folglich wird im Rahmen des Schrittes c) ein Längendifferenz-Datensatz berechnet, der sich aus der Differenz der definierten Längenabmessung der vorgefertigten Zahnrohlinge und einer Einsetztiefe der wenigstens zwei vorgefertigten Zahnrohlinge in dem Basiselement ergibt. Der Längendifferenz-Datensatz beschreibt somit den Unterschied zwischen dem "Ist" der vorgefertigten Zahnrohlinge und dem "Soll", welches diese beim Einsetzen in das Basiselement aufweisen sollen.
Wie dargelegt, findet im Schritt b) eine Auswahl von digitalen Darstellungen von vorgefertigten Zahnrohlingen statt. Diese Auswahl aus einer Menge von digitalen Darstellungen von vorgefertigten Zahnrohlingen kann erfolgen, um die kleinstmögliche Abweichung einer geometrischen Form der vorgefertigten Zahnrohlinge von der tatsächlich im Mund des Patienten auftretenden Zahnsituation zu erzielen. Dies ist dann der Fall, wenn die noch vorhandenen Zähne dem Patienten entnommen werden, um anstelle dieser die einzusetzenden Zahnrohlinge zu nutzen. Falls der Patient keinerlei Zähne mehr aufweist, muss es das Ziel sein, die digitalen Darstellungen der vorgefertigten Zahnrohlinge so auszuwählen, dass eine kleinstmögliche Abweichung einer geometrischen Form der vorgefertigten Zahnrohlinge von einer im Vorhinein vordefinierten Zahnanordnung erreicht wird. Es wird folglich jeweils ein Zahnrohling ausgewählt, der optimal in das Gesamtbild des zu erstellenden Gebisses passt. Dabei werden Größe, geometrische Form und Abstand bzw. Platzierung zu seinen Nachbarn ausschlaggebend sein. Das erfindungsgemäße Verfahren zeichnet sich weiterhin durch einen Schritt d) aus, im Rahmen dessen ein digitales Bild der Negativform aufgebaut wird. Dieses digitale Bild dient als Grundlage, auf Basis dessen später die tatsächliche Negativform mittels einer Maschine erstellt wird (vgl. Schritt e)). Dabei ist vorgesehen, dass die Negativform die Position der wenigstens zwei vorgefertigten Zahnrohlinge auf dem ebenfalls noch zu erstellenden Basiselement codiert. Die Negativform definiert also die Position der vorgefertigten Zahnrohlinge relativ zueinander und in Bezug zu dem Element oder den Elementen des Basiselementes. Im Rahmen eines Schrittes e) wird die Negativform maschinell erstellt. Dieses maschinelle Erstellen geschieht mittels der digitalen Informationen des digitalen Bildes der Negativform. Ein entsprechendes Erstellen kann durch abtragende oder aufbauende, computergestützte Herstellungsverfahren erfolgen. In diesem Zusammenhang seien insbesondere die folgenden Herstellungsverfahren erwähnt: Fräsen, Fräsen via CAD/CAM, Drehen, Rapid Prototyping, 3D-Laserlithographie, 3D-Lithografie, SLM (selektives Laserschmelzen), 3D-Stereolithgrafie, 3D-Inkjet, FDM (fused deposition modelling) oder 3D-Laserlithographie.. Erfindungsgemäß ist im Rahmen des Schrittes g) ein maschinelles Verkürzen der Längenabmessungen der vorgefertigten Zahnrohlinge von einer Basalseite aus vorgesehen. Nach dem Erstellen der Negativform werden die schon vorhandenen vorgefertigten Zahnrohlinge im Rahmen des Schrittes f) in die Negativform eingeführt. Nunmehr dient die Negativform als Halterung für die vorgefertigten Zahnrohlinge. Da die Negativform aufgrund des angestrebten digitalen Bildes des zu erstellenden künstlichen Gebisses hergestellt ist, codiert die Negativform gleichzeitig die Anordnung, Länge und Höhe der zu nutzenden Zahnrohlinge. Werden diese nun eingebracht, ist es einfach möglich, sie maschinell zu verkürzen. Dieses Verkürzen auf der basalen Seite geschieht auf Basis des Längendifferenz-Datensatzes. Folglich werden die vorgefertigten Zahnrohlinge genau um jenen Längenanteil gekürzt, um welchen sie die angestrebte Länge überragen. Anschließend erfolgt im Rahmen des Schrittes h) nur noch ein Verbinden des Basiselements und der verkürzten Zahnrohlinge zu dem künstlichen Gebiss.
In einer weiteren Ausführungsvariante des erfindungsgemäßen Verfahrens ist vorgesehen, dass das digitale Modell des künstlichen Gebisses computergestützt in zwei Teile aufgeteilt wird: ein digitales Abbild des Basiselements und ein digitales Ebenbild der wenigstens zwei Zahnrohlinge. Ziel ist dabei, dass auch das Basiselement computergestützt entworfen wird. Insbesondere ermöglicht diese Variante eine sehr gute Anpassung des Basiselements und der Zahnrohlinge bzw. der digitalen Darstellungen der vorgefertigten Zahnrohlinge. Darüber hinaus können in das digitale Abbild des Basiselements digitale Modelle von Befestigungsmitteln etc. eingebracht werden. Weiterhin wird die digitale Darstellung der vorgefertigten Zahnrohlinge in ihrer Position relativ zu dem Basiselement angepasst. Diese Anpassung einer Höhenposition der digitalen Darstellung der vorgefertigten Zahnrohlinge ermöglicht die Konstruktion des digitalen Modells des künstlichen Gebisses am Rechner. Auch wird durch die Anpassung der Höhenposition der Längendifferenz-Datensatz berechnet, da die Anpassung der Höhenposition Rückschlüsse auf jene Tiefe gibt, mit der die vorgefertigten Zahnrohlinge später in das Basiselement eingesetzt werden sollen.

Auf Basis des digitalen Abbildes des Basiselementes ist ein maschinelles Fertigen des eigentlichen Basiselementes möglich. Dazu können sowohl abtragende, als auch auftragende computergestützte Herstellungsverfahren genutzt werden. So ist ein Fräsen, Drehen oder Sägen für ein abtragendes Herstellungsverfahren nutzbar. Die schon genannten auftragenden Herstellungsverfahren, welche bei der Herstellung der Negativform Verwendung finden, können auch für die Herstellung des Basiselements eingesetzt werden.

Weitere Maßnahmen und Vorteile der Erfindung ergeben sich aus den Ansprüchen, der nachfolgenden Beschreibung und den Zeichnungen. In den Zeichnungen ist die Erfindung in mehreren Ausführungsbeispielen dargestellt. Gleiche oder funktionsgleiche oder hinsichtlich ihrer Funktionen einander entsprechende Elemente werden dabei mit gleichen Bezugsziffern bezeichnet. Die Erfindung ist nicht auf die Ausführungsbeispiele beschränkt.

Es zeigen:
- Figur 1: ein Flussdiagramm des erfindungsgemäßen Verfahrens,
- Figur 2: ein Auswählen von digitalen Darstellungen von vorgefertigten Zahnrohlingen,
- Figur 3: eine Berechnung eines Längendifferenzdatensatzes,
- Figur 4: ein Einfügen der vorgefertigten Zahnrohlinge in eine Negativform,
- Figuren 5 und 6: ein maschinelles Verkürzen der Längenabmessung der vorgefertigten Zahnrohlinge,
- Figur 7: die verkürzten Zahnrohlinge, und
- Figur 8: die Verbinden des Basiselementes und der verkürzten Zahnrohlinge zu dem künstlichen Gebiss.

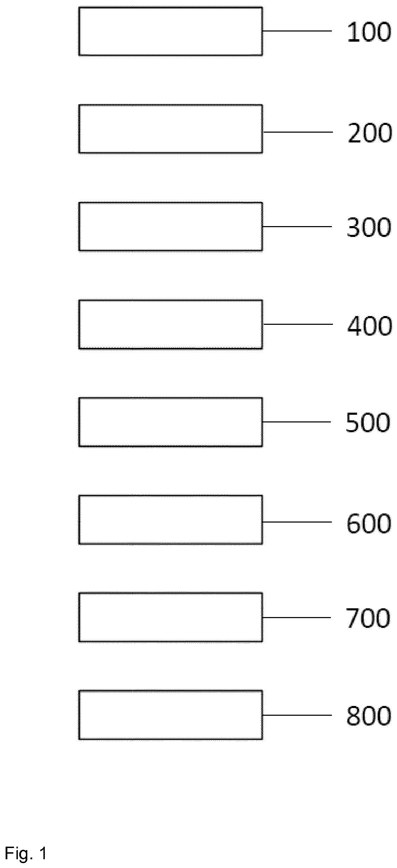

Ausgangspunkt für das erfindungsgemäße Verfahren ist der Gedanke der Erstellung einer Negativform 50, welche dazu dient, eine Mehrzahl von vorgefertigten Zahnrohlingen 40,40',40" zu halten. Dabei ist die Negativform 50 derart ausgestaltet, dass in ihr die Zahnrohlinge 40,40',40" so positioniert werden, wie sie tatsächlich im späteren künstlichen Gebiss 10 auch angeordnet sein sollen. Die vorkonfektionierten Zahnrohlinge 40,40',40" weisen dabei Längen auf, die nicht jenen entsprechen, wie sie tatsächlich im noch herzustellenden künstlichen Gebiss 10 benötigt werden. Durch die Halterung in der Negativform 50 ist aber ein präzises Verkürzen der vorgefertigten Zahnrohlinge 40,40',40" möglich. Darüber hinaus weist das erfindungsgemäße Verfahren die Besonderheit auf, dass die Negativform 50 mittels eines Rechners aus einem digitalen Abbild einer Zahnsituation 15 errechnet wird. Das Flussdiagramm in Figur 1 soll die verschiedenen Verfahrensschritte verdeutlichen.

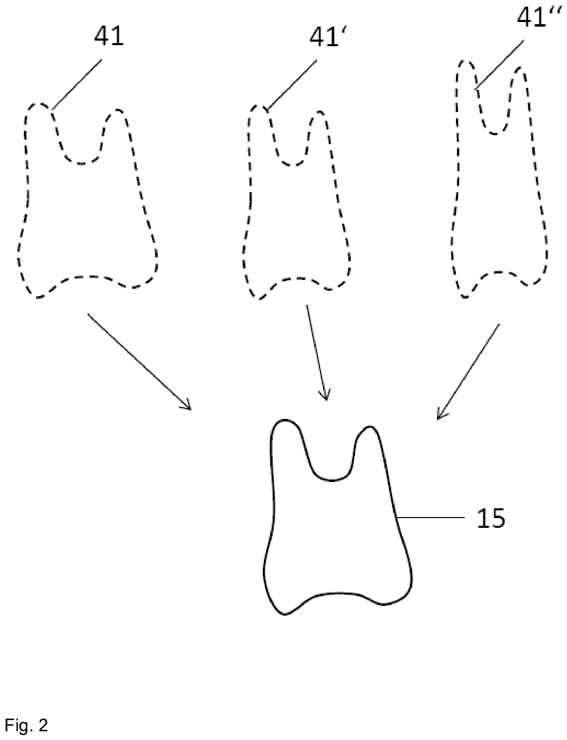

In Schritt 100 wird ein digitales Abbild der vorliegenden Zahnsituation 15 des Patienten erstellt. Dies kann vorzugsweise mittels eines Interoralscanners geschehen. Im Anschluss wird auf Basis des digitalen Abbildes ein digitales Modell 11 des zu erstellenden künstlichen Gebisses 10 angefertigt. Soll beispielsweise eine Vollprothese erstellt werden, wird im Rahmen des digitalen Abbildes der Zahnsituation nur ein Abbild des Zahnfleisches generiert. Ein Zahntechniker muss dann, basierend auf diesem Abbild des Zahnfleisches, das später einzusetzende künstliche Gebiss 15 digital erstellen. Erfindungsgemäß kann der Zahntechniker sich dazu digitaler Darstellungen 41,41',41" von vorgefertigten Zahnrohlingen 40,40',40" bedienen. Im Rahmen des Schrittes 200 wird das digitale Modell 11 des künstlichen Gebisses 10 erstellt. Beispielhaft verdeutlicht die Figur 2, dass eine Auswahl vorgenommen wird, welche der digitalen Darstellungen 41,41',41" eines vorgefertigten Zahnrohlings 40,40',40" am ehesten der angestrebten Zahnsituation 15 entspricht. Dabei finden als Auswahlkriterium insbesondere Größe, Länge und Volumen Verwendung.

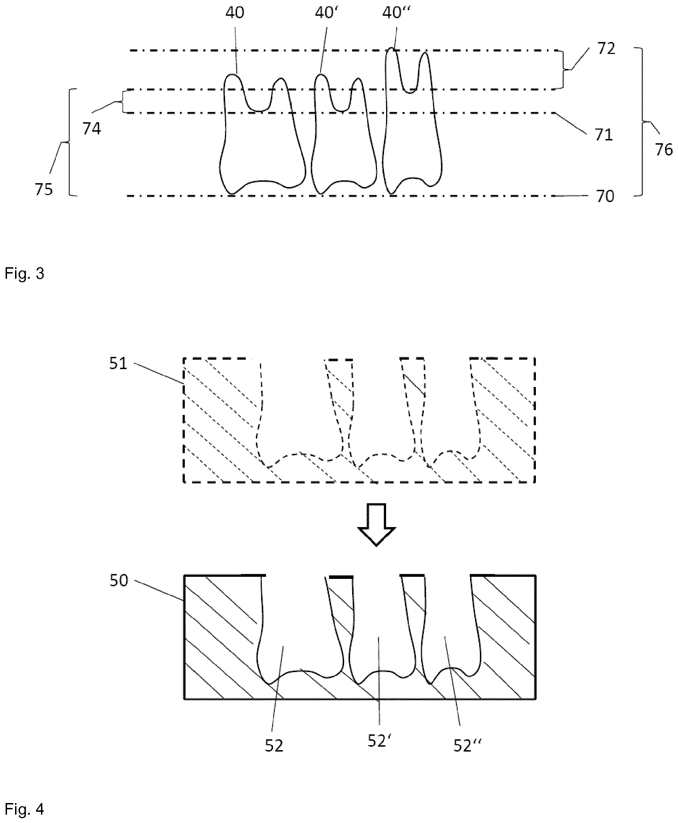

Nachdem im Schritt 200 die Form des digitalen Modells 11 berechnet und eine Auswahl der digitalen Darstellungen 41,41',41" der vorgefertigten Zahnrohlinge 40,40',40" stattgefunden hat, wird in Schritt 300 ein Längendifferenz-Datensatz 72 bestimmt. Dies soll die Figur 3 verdeutlichen. Aufgrund unterschiedlicher anatomischer Gegebenheiten sind die Längen der Zähne bei jedem Menschen unterschiedlich. Die vorgefertigten Zahnrohlinge 40,40',40" weisen allerdings vordefinierte Längen auf. Darüber hinaus ist bekannt, wie tief die Zahnrohlinge in das Basiselement für eine stabile und nicht reversible Montage eingesetzt werden müssen. Diese als Einsetztiefe 74 bezeichnete Tiefe ist vordefiniert und abhängig von der Dimension des Basiselements 20. Der Abstand zwischen einer Basislinie 70, auf welcher die Schneidfläche der Zähne angeordnet ist, und einer Kapitelllinie 71 definiert die sichtbare Länge der vorgefertigten Zahnrohlinge 40,40',40". Daran schließt sich die Einsetztiefe 74 an. Aus beiden Längen ergibt sich die gewünschte Zahnlänge 75. Von dieser gewünschten Zahnlänge 75 weicht beispielhaft in Figur 3 der vorgefertigte Zahnrohling 40" mit seiner tatsächlichen Zahnlänge 76 signifikant ab. Der Längendifferenz-Datensatz 72 aus der gewünschten Zahnlänge 75 von der tatsächlichen Zahnlänge 76 wird im Rahmen des Schrittes 300 berechnet.

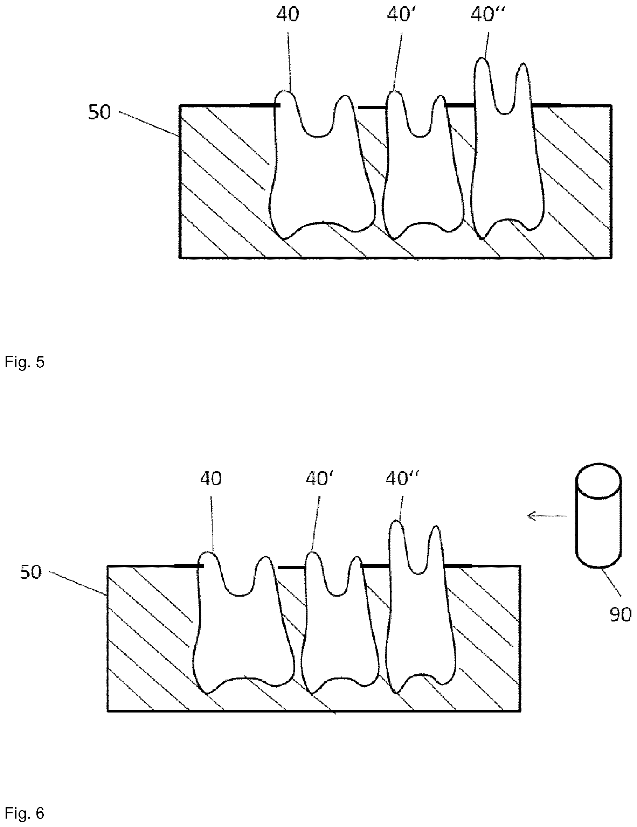

Im Rahmen des Schrittes 400 werden ausgehend von dem Längendifferenz-Datensatz 72 sowie weiterer Informationen über die äußere Gestalt der vorgefertigten Zahnrohlinge 40,40',40" und dem digitalen Abbild 11 der Zahnsituation ein digitales Bild 51 der Negativform 50 geschaffen. Dies verdeutlicht auch die Figur 4. Das digitale Bild 51 ermöglicht eine maschinelle Erstellung 500 der Negativform 50. Die maschinelle Erstellung 500 geschieht dabei vorzugsweise mit auftragenden und computergestützten Herstellungsverfahren wie Rapid Prototyping. In die so erstellte Negativform 50 werden im Rahmen des Schrittes 600 die vorgefertigten Zahnrohlinge 40 eingefügt, wie es die Figur 5 darstellt.

Die erfindungsgemäße Negativform 50 hält die vorgefertigten Zahnrohlinge 40,40',40" in einer Art und Weise, dass jene die Negativform 50 an ihrer basalen Seite überragen. In Schritt 700 findet ein maschinelles Verkürzen 90 der vorgefertigten Zahnrohlinge mittels einer Fräse etc. statt. Dabei werden die vorgefertigten Zahnrohlinge 40,40',40" um den Längendifferenz-Datensatz 72 gekürzt. Wie insbesondere der Figur 6 zu entnehmen ist, ragen die vorgefertigten Zahnrohlinge 40,40',40" um jenen Längendifferenz-Datensatz 72 über die Negativform 50 heraus. Somit ist ein einfaches Abtragen der überstehenden Längen möglich. Gleichzeitig schützt die Negativform 50 die Außenseiten der später in das Gebiss einzusetzenden Anteile der vorgefertigten Zahnrohlinge 40,40',40". Auch sorgt die Negativform 50 für eine mechanische Stabilität und Halterung der vorgefertigten Zahnrohlinge 40,40',40" während des Verkürzungsvorganges im Rahmen des Schrittes 700. Dadurch kann eine Präzision in der Kürzung der vorgefertigten Zahnrohlinge auf +/- 20 µm, insbesondere +/- 10 µm erreicht werden. Diese hohe Präzision ist Voraussetzung für die Erschaffung eines künstlichen Gebisses, 10 welches von dem Patienten ohne lange Gewöhnungsphase genutzt werden kann.

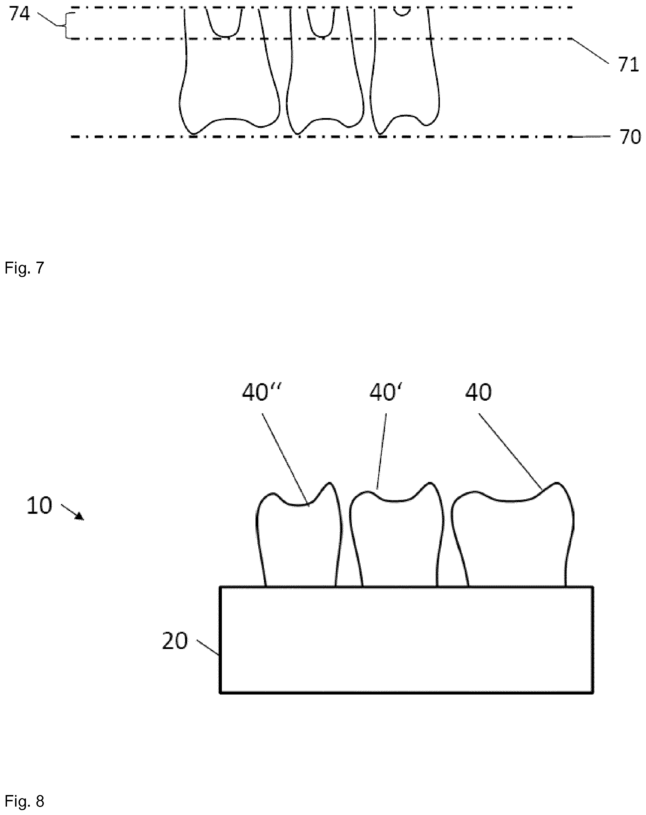

In Figur 7 sind die verkürzten Zahnrohlinge zu sehen. Jene werden im Schritt 800 mit einem Basiselement 20 verbunden, um das in Figur 8 dargestellte künstliche Gebiss 10 zu erzeugen. Das künstliche Gebiss 10 besteht somit aus dem Basiselement 20, in welchem die verkürzten Zahnrohlinge 40,40',40" angeordnet sind.

### Bezugszeichen:

- 10: künstliches Gebiss
- 11: digitales Modell des künstlichen Gebisses
- 15: Zahn oder Zahnsituation oder vordefinierten Zahnanordnung

- 20: Basiselement

- 40,40',40": vorgefertigte Zahnrohlinge
- 41,41',41": digitale Darstellung von vorgefertigten Zahnrohlingen

- 50: Negativform
- 51: digitales Bild der Negativform
- 52: Aufnahmen für Zahnrohlinge in Negativform

- 70: Basislinie
- 71: Kapitelllinie
- 72: Längendifferenz-Datensatz
- 74: Einsetztiefe
- 75: Zahnlänge
- 76: tatsächliche Zahnlänge des Zahnrohlings 40"

- 90: maschinelles Verkürzen

- 100: Erstellen eines digitalen Abbildes
- 200: Formen eines digitalen Modells
- 300: Berechnung eines Längendifferenzdatensatzes
- 400: Aufbauen eines digitalen Bildes einer Negativform
- 500: maschinelles Erstellen der Negativform
- 600: Einfügen der vorgefertigten Zahnrohlingen in die Negativform
- 700: maschinelles Verkürzen der vorgefertigten Zahnrohlinge
- 800: Verbinden eines Basiselementes und der verkürzten Zahnrohlinge

## Patentansprüche

1. Verfahren zum Herstellen eines künstlichen Gebisses (10), wobei das künstliche Gebiss ein Basiselement (20) aufweist, weiches wenigstens zwei Zahnrohlinge (40,40',40") hält, mit den Schritten:
a. Erstellen (100) des digitalen Abbildes einer Zahnsituation,
b. computergestütztes Erzeugern (200) eines digitalen Modelles des künstlichen Gebisses auf Basis des digitalen Abbildes,
∘ unter Verwendung und Auswahl von digitalen Darstellungen (41,41',41") von vorgefertigten Zahnrohlingen,
∘ wobei die vorgefertigten Zahnrohlinge eine definierte Längenabmessung aufweisen,
c. Berechnung (300) eines Längendifferenz-Datensatzes, erstellt aus der definierten Längenabmessung und einer Einsetztiefe (74) der wenigstens zwei vorgefertigten Zahnrohlinge (40,40',40") in dem Basiselement (20),
d. Aufbauen (400) eines digitalen Bildes (51) einer Negativform (50), bei der die wenigstens zwei vorgefertigten Zahnrohlinge das Positiv bilden, wobei die Negativform (50) die Position der wenigstens zwei vorgefertigten Zahnrohlinge (40,40',40") auf dem Basiselement (20) codiert,
e. Maschinelles Erstellen (500) der Negativform (50) mittels des digitalen Bildes (51) der Negativform,
f. Einfügen (600) der vorgefertigten Zahnrohlinge (40,40',40") in die Negativform (20),
g. computergestütztes Maschinelles Verkürzen (700) der Längenabmessung der vorgefertigten Zahnrohlinge (40,40',40") von einer basalen Seite auf Basis des Längendifferenz-Datensatzes (72) zur Herstellung der verkürzten Zahnrohlinge,
h. Verbinden (800) des Basiselementes (20) und der verkürzten Zahnrohlinge zu dem künstlichen Gebiss (10).

2. Verfahren nach Anspruch 1, **dadurch gekennzeichnet, dass** zum Erstellen der Negativform ein auftragendes computergestütztes Herstellungsverfahren genutzt wird, insbesondere, dass das auftragende computergestützte Herstellungsverfahren ausgewählt ist aus der Gruppe bestehend aus: Rapid Prototyping, 3D-Lithografie, SLM (selektives Laserschmelzen), 3D-Stereolithgrafie, 3D-Inkjet, FDM (fused deposition modelling) und 3D-Laserlithographie oder mindestens zwei davon.

3. Verfahren nach wenigstens einem der vorherigen Ansprüche, **dadurch gekennzeichnet, dass** die vorgefertigten Zahnrohlinge kraft- und/oder form- und/oder stoffschlüssig in der Negativform gehalten werden.

4. Verfahren nach wenigstens einem der vorherigen Ansprüche, **dadurch gekennzeichnet, dass** das Verfahren den Schritt aufweist:
• Erstellen eines Befestigungsmittels zur Verbindung der verkürzten Zahnrohlinge mit dem Basisteil, wobei das Befestigungsmittel ein erstes Codierungsmittel aufweist, welches eine räumlich eindeutige Positionierung des verkürzten Zahnrohlings in dem Basisteil ermöglicht.

5. Verfahren nach wenigstens einem der vorherigen Ansprüche, **dadurch gekennzeichnet, dass** die Negativformen mit wenigstens einem zweiten Codierungsmittel versehen werden, welches eine räumlich eindeutige Positionierung des vorgefertigten Zahnrohlings in der Negativform ermöglicht.

6. Verfahren nach Anspruch 1, **dadurch gekennzeichnet, dass** In Schritt c) ausgehend von der Zahnsituation und/oder einer vordefinierten Zahnanordnung die ausgewählten, digitalen Darstellungen der Zahnrohlinge an einer Kronenseite entlang einer Zahnlinie angeordnet werden.

7. Verfahren nach einem der Ansprüche 1 oder 6, **dadurch gekennzeichnet, dass** nach dem Schritt b) das Verfahren den Schritt aufweist: Anpassen einer Höhenposition der digitalen Darstellungen der vorgefertigten Zahnrohlinge in dem digitalen Modell des künstlichen Gebisses.

8. Verfahren nach wenigstens einem der vorherigen Ansprüche 1 bis 7, **dadurch gekennzeichnet, dass** das Verfahren den Schritt aufweist: Computergestütztes Teilen des digitalen Modelles des künstlichen Gebisses in ein digitales Abbild des Basiselementes und ein digitales Ebenbild der wenigstens zwei Zahnrohlinge.

9. Verfahren nach Anspruch 8, **dadurch gekennzeichnet, dass** das Verfahren den Schritt aufweist: Maschinelles Fertigen des Basiselementes auf Basis des digitalen Abbildes des Basiselementes.

10. Verfahren nach wenigstens einem der vorherigen Ansprüche 1 oder 9, **dadurch gekennzeichnet, dass** ein abtragendes und/oder auftragendes computergestütztes Herstellungsverfahren genutzt wird in wenigstens einem der Verfahrensschritte, ausgewählt aus der Gruppe bestehend aus: Maschinelles Erstellen der Negativform und maschinelles Fertigen des Basiselementes.

11. Verfahren nach wenigstens einem der vorherigen Ansprüche, **dadurch gekennzeichnet, dass** zum Formen des digitalen Modelles des künstlichen Gebisses ein digitalen Abbildes einer Zahnsituation verwendet wird, das die Form eines Zahnfleischareals eines Patienten wiedergibt.

12. Computerlesbarer Datenträger, der Instruktionen umfasst, die einen Computer veranlassen, ein Verfahren nach einem der Ansprüche 1 bis 11 auszuführen.

13. Computer mit einem computerlesbaren Datenträger nach Anspruch 12.

## Claims

1. Method for producing a denture (10), wherein the denture comprises a base element (20), which supports at least two tooth blanks (40,40',40"), comprising the steps:
a. preparing (100) the digital image of a tooth situation,
b. computer-based generating (200) a digital model of the denture based on the digital image, by means of using and selecting digital representations (41,41',41") of pre-fabricated tooth blanks, wherein the pre-fabricated tooth blanks have a defined length dimension,
c. calculation (300) of a length difference data set prepared from the defined length dimension and an insertion depth (74) of the at least two pre-fabricated tooth blanks (40,40',40") in the base element (20),
d. building-up (400) a digital image (51) of a negative mould (50), in which the at least two pre-fabricated tooth blanks form the positive, wherein the negative mould (50) encodes the position of the at least two pre-fabricated tooth blanks (40,40',40") on the base element (20),
e. preparing by machining (500) the negative mould (50) by means of the digital image (51) of the negative mould,
f. inserting (600) the pre-fabricated tooth blanks (40,40',40") into the negative mould (20),
g. computer-based shortening by machining (700) the length dimension of the pre-fabricated tooth blanks (40,40',40") from a basal side based on the length difference data set (72) in order to produce the shortened tooth blanks,
h. connecting (800) the base element (20) and the shortened tooth blanks to form the denture (10).

2. Method according to claim 1, **characterised in that**, for preparing the negative mould, a computer-based up-building production method is used, in particular **in that** the computer-based up-building production method is selected from the group consisting of: rapid prototyping, 3D lithography, SLM (selective laser melting), 3D stereolithography, 3D inkjet, FDM (fused deposition modelling), and 3D laser lithography, or at least two thereof.

3. Method according to at least one of the preceding claims, **characterised in that** the pre-fabricated tooth blanks are supported in the negative mould in a non positive fit and/or form closure and/or material bonded manner.

4. Method according to at least one of the preceding claims, **characterised in that** the method comprises the step of:
preparing an attachment means for connecting the shortened tooth blanks to the base element, wherein the attachment means comprises a first coding means that facilitates definite spatial positioning of the shortened tooth blank in said base part.

5. Method according to at least one of the preceding claims, **characterised in that** the negative moulds are provided with at least one second coding means that facilitates definite spatial positioning of the pre-fabricated tooth blank in the negative mould.

6. Method according to claim 1, **characterised in that**, in step c), the selected digital representations of the tooth blanks are arranged on a crown side along a tooth line based on the tooth situation and/or a pre-defined tooth arrangement.

7. Method according to any one of the claims 1 or 6, **characterised in that** the method comprises, after step b), the step of: adapting a height position of the digital representations of the pre-fabricated tooth blanks in the digital model of the denture.

8. Method according to at least one of the preceding claims 1 to 7, **characterised in that** the method comprises the step of: computer-based splitting of the digital model of the denture into a digital image of the base element and a digital image of the at least two tooth blanks.

9. Method according to claim 8, **characterised in that** the method comprises the step of: fabricating, by machining, the base element based on the digital image of the base element.

10. Method according to at least one of the preceding claims 1 or 9, **characterised in that** an ablating and/or up-building computer-based production method is used in at least one of the procedural steps of the method, selected from the group consisting of: preparing the negative mould by machining and fabricating the base element by machining.

11. Method according to at least one of the preceding claims, **characterised in that** a digital image of a tooth situation that reflects the shape of a gingival area of a patient is used for shaping the digital model of the denture.

12. Computer-readable data medium that comprises instructions that trigger a computer to run a method according to any one of the claims 1 to 11.

13. Computer with a computer-readable data medium according to claim 12.

## Revendications

1. Procédé pour produisant un dentier (10), selon lequel le dentier comprend un élément de base (20), qui soutient au moins deux ébauches de dent (40,40',40"), comprenant les étapes :
a. préparant (100) l'image numérique d'une situation de dent,
b. générant assisté par ordinateur (200) un modèle numérique du dentier sur la base de l'image numérique, au moyen d'utilisant et sélectionnant des représentations numériques (41,41',41") des ébauches de dent préfabriquées, selon lequel les ébauches de dent préfabriquées ont une mesure de longueurs définie,
c. le calcul (300) d'un ensemble de données de la différence de longueurs, préparé à partir de la mesure de longueurs définie et une profondeur d'insertion (74) des au moins deux ébauches de dent préfabriqué (40,40',40") dans l'élément de base (20),
d. constituant (400) une image numérique (51) d'un moule négatif (50), dans laquelle les au moins deux ébauches de dents préfabriquée forment le positif, selon lequel le moule négatif (50) encode la position des au moins deux ébauches de dent préfabriquées (40,40',40") sur l'élément de base (20),
e. préparant par une machine (500) le moule négatif (50) au moyen de l'image numérique (51) du moule négatif,
f. insérant (600) les ébauches de dent préfabriquées (40,40',40") dans le moule négatif (20),
g. raccourcissant assisté par ordinateur par une machine (700) la mesure de longueurs des ébauches de dent (40,40',40") à partir d'un côté basal sur la base de l'ensemble de données de la différence de longueurs (72) pour produire les ébauches de dent raccourcies,
h. connectant (800) l'élément de base (20) et les ébauches de dent raccourcies pour former le dentier (10).

2. Procédé selon la revendication 1, **caractérisé en ce que** pour préparant le moule négatif, une méthode de production d'ajout assistée par ordinateur est utilisée, en particulière **en ce que** la méthode de production d'ajout assistée par ordinateur est sélectionnée à partir du groupe consistant en : le prototypage rapide, la lithographie en 3D, SLM (la fusion sélective par laser), la stéréolithographie en 3D, le jet d'encre en 3D, FDM (la modélisation par dépôt fusionné), et la lithographie laser en 3D, ou au moins deux desquelles.

3. Procédé selon au moins une des revendications précédentes, **caractérisé en ce que** les ébauches de dent préfabriquées sont soutenues dans le moule négatif par liaison par complémentarité de force et/ou de forme et/ou de matière.

4. Procédé selon au moins une des revendications précédentes, **caractérisé en ce que** le procédé comprend l'étape :
préparant un moyen de fixation pour connectant les ébauches de dent raccourcies à l'élément de base, selon lequel le moyen de fixation comprend un premier moyen de codage qui facilite le positionnement spatial défini de l'ébauche de dent raccourcie dans l'élément de base.

5. Procédé selon au moins une des revendications précédentes, **caractérisé en ce que** le moule négatif est prévu avec au moins un deuxième moyen de codage qui facilite le positionnement spatial défini de l'ébauche de dent préfabriquée dans le moule négatif.

6. Procédé selon la revendication 1, **caractérisé en ce que**, à l'étape c), les représentations numériques des ébauches de dent sont arrangées à un côté de couronnes le long d'une ligne de dent basé sur la situation de dent et/ou un arrangement de dent prédéfinie.

7. Procédé selon l'une des revendications 1 ou 6, **caractérisé en ce que** le procédé comprend, après l'étape b), l'étape de : adaptant une position d'hauteur des représentations numériques des ébauches de dent préfabriquées dans le modèle numérique du dentier.

8. Procédé selon au moins une des revendications précédentes 1 à 7, **caractérisé en ce que** le procédé comprend l'étape de : fractionnant assisté par ordinateur du modèle numérique du dentier en une image numérique de l'élément de base et une image numérique des au moins deux ébauches de dent.

9. Procédé selon la revendication 8, **caractérisé en ce que** le procédé comprend l'étape : fabriquant par une machine l'élément de base sur la base de l'image numérique de l'élément de base.

10. Procédé selon au moins une des revendications précédentes 1 ou 9, **caractérisé en ce qu'** une méthode de production d'ablation et/ou d'ajout assistée par ordinateur est utilisée en au moins une des étapes de procédé, sélectionnée à partir du groupe consistant en : préparant par une machine le moule négatif et fabriquant par une machine l'élément de base.

11. Procédé selon au moins une des revendications précédentes, **caractérisé en ce qu'** une image numérique d'une situation de dent qui reflète la forme de la région de gencive d'un patient est utilisée pour formant le modèle numérique du dentier.

12. Un support des données lisible par ordinateur qui comprend des instructions à exécuter un procédé selon l'une des revendications 1 à 11.

13. Un ordinateur avec un support des données lisible par ordinateur selon la revendication 12.
